(19) Europäisches Patentamt
European Patent Office
Office européen des brevets

(11) Veröffentlichungsnummer: **0 235 532**
**B1**

(12) # EUROPÄISCHE PATENTSCHRIFT

(45) Veröffentlichungstag der Patentschrift: **14.06.89**

(51) Int. Cl.⁴: **C07C 47/277**, C07D 319/06

(21) Anmeldenummer: **87100771.2**

(22) Anmeldetag: **21.01.87**

(54) **Verfahren zur Herstellung von 4-Monoacetalen des 2-Methyl-2-buten-1,4-dials.**

(30) Priorität: **25.01.86 DE 3602253**

(43) Veröffentlichungstag der Anmeldung:
**09.09.87 Patentblatt 87/37**

(45) Bekanntmachung des Hinweises auf die Patenterteilung:
**14.06.89 Patentblatt 89/24**

(84) Benannte Vertragsstaaten:
**CH DE FR GB LI NL**

(56) Entgegenhaltungen:
**EP-B- 0 009 752**
**EP-B- 0 090 231**
**DE-A- 2 357 752**
**DE-A- 2 357 810**
**DE-A- 2 513 999**
**DE-B- 2 365 421**
**DE-C- 2 225 612**

(73) Patentinhaber: **BASF Aktiengesellschaft,**
**Carl-Bosch-Strasse 38, D-6700 Ludwigshafen(DE)**

(72) Erfinder: **Fischer, Rolf, Dr., Bergstrasse 98,**
**D-6900 Heidelberg(DE)**
Erfinder: **Schulz, Bernhard, Dr., Kurpfalzring 28,**
**D-6830 Schwetzingen(DE)**

**EP 0 235 532 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft ein Verfahren zur Herstellung von 4-Monoacetalen des (E)-2-Methyl-2-buten-1,4-dials (2-Methyl-fumardialdehyds) durch Umsetzung von 2-Methyl-2-buten-1,4-dial, dessen 1-Monoacetalen oder 1,4-Bisacetalen mit Wasser und/oder Alkoholen in Gegenwart von sauren Verbindungen.

4-Monoacetale des (E)-2-Methyl-2-buten-1,4-dials sind wertvolle Bausteine für die Synthese verschiedener Terpene mit biologischer und pharmakologischer Wirksamkeit. Für ihre Herstellung sind schon mehrere Verfahren beschrieben worden. So ist aus der DE-OS 23 57 752 und der DE-OS 23 57 810 bekannt, daß sich Acetale bzw. Acylale des 3-Methyl-2-butenals mit Selendioxid zu 4-Acetalen des (E)-2-Methyl-2-buten-1,4-dials oxidieren lassen. Nach dem in der DE-OS 22 25 612 beschriebenen Verfahren lassen sich cyclische Acetale des 3-Methyl-2-buten-4-ol-1-als mit schwefelsaurer Chromsäurelösung zu den entsprechenden 4-Acetalen des 2-Methyl-2-buten-1,4-dials oxidieren.

(E)-2-Methyl-2-buten-1,4-dial-4-acetale können auch nach den Angaben der DE-OS 25 13 999 in einem vielstufigen Verfahren hergestellt werden, wobei man in einem ersten Schritt Crotonaldehydacetale einer Ozonolyse unterwirft. In der EP-PS 9 752 wird die Umsetzung von Sechsringacetalen des 3-Methyl-3-buten-1-als mit Nitrosierungsmitteln wie Nitrosylchlorid oder Salpetrigsäureestern in Gegenwart von Methanol und Salzsäure beschrieben. Aus den dabei gebildeten 2-Chlor-2-methylbutan-1,4-dial-bisacetalen spaltet man mit Basen Chlorwasserstoff ab.

Die so erhaltenen Bisacetale des 2-Methyl-2-buten-1,4-dials, in denen in 1-Stellung eine Dialkylacetal-, in 4-Stellung eine cyclische Acetalgruppe gebunden ist, lassen sich mit verdünnten wäßrigen Mineralsäuren selektiv zum 4-Acetal des 2-Methyl-furmardialdehyds verseifen.

Die Verfahren nach dem Stand der Technik sind für die technische Herstellung der begehrten 2-Methyl-fumardialdehyd-4-monoacetale nicht voll befriedigend, da sie entweder zu aufwendig sind oder aber teurer und/oder toxischer Oxidationsmittel, wie Selendioxid, Chromsäure, Ozon, Nitrosylchlorid oder Salpetrigsäureester bedürfen.

Es war daher die Aufgabe der Erfindung, ein Verfahren zu ihrer Herstellung zu entwickeln, das es erlaubt die 4-Monoacetale des 2-Methyl-2-buten-1,4-dials ausgehend von gut zugänglichen Ausgangsverbindungen auf möglichst einfache Weise herzustellen und das die Nachteile der bekannten Verfahren nicht aufweist.

Gegenstand der Erfindung ist ein Verfahren zur Herstellung von 4-Monoacetalen des (E)-2-Methyl-2-buten-1,4-dials der allgemeinen Formel I

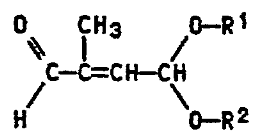

(I),

in der $R^1$ und $R^2$ einen aliphatischen Kohlenwasserstoffrest mit 1 bis 12, vorzugsweise 1 bis 4 C-Atomen, einen Cycloalkylrest mit 5 bis 7 C-Atomen oder eine Benzylgruppe bedeuten, oder aber $R^1$ und $R^2$ zusammen für einen Ethylen- oder Propylenrest stehen, die noch durch Alkylgruppen mit 1 bis 4 C-Atomen, vorzugsweise Methylgruppen, substituiert sein können, das dadurch gekennzeichnet ist, daß man Verbindungen der allgemeinen Formel II

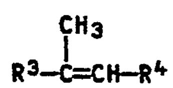

(II),

in der $R^3$ und $R^4$ entweder beide für eine der Gruppen

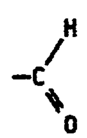

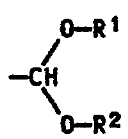

oder

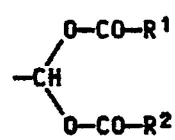

stehen, in der $R^1$ und $R^2$ die oben angegebene Bedeutung haben, oder aber $R^3$ eine der Gruppen

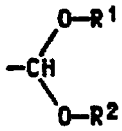 oder 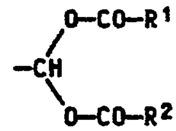

und $R^4$ die Formylgruppe 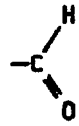

bedeuten, bei Temperaturen von 20 bis 200°C, vorzugsweise 40 bis 150°C in Gegenwart von sauren Verbindungen mit Verbindungen der Formel III

$R^5$-OH (III),

in der $R^5$ Wasserstoff oder einen Alkylrest mit 1 bis 12 C-Atomen, vorzugsweise 1 bis 4 C-Atomen, insbesondere eine Methylgruppe oder einen Hydroxyalkylrest mit 2 bis 5 C-Atomen, bedeutet, umsetzt, wobei für den Fall, das $R^3$ und $R^4$ für

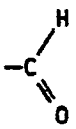 stehen, $R^5$ einen Alkylrest bedeutet und für den Fall, daß $R^3$ und $R^4$ für

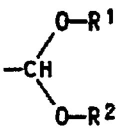 oder 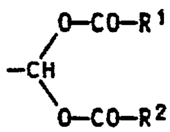 stehen, $R^5$ Wasserstoff bedeutet und

für den Fall, daß $R^3$ für 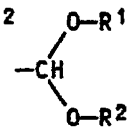 oder 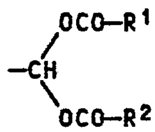 und $R^4$ für

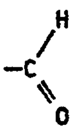 stehen,

$R^5$ Wasserstoff und/oder einen Alkylrest bedeutet.

Praktisch bedeutet das im wesentlichen, daß man zur Herstellung von 4-Monoacetalen des (E)-2-Methyl-2-buten-1,4-dials der allgemeinen Formel I ein 1,4-Bisacetal der Formel II in Gegenwart von sauren Verbindungen mit Wasser, 2-Methyl-2-buten-1,4-dial in Gegenwart von sauren Verbindungen mit Alkanolen oder 1-Monoacetale des (E)-2-Methyl-2-buten-1,4-dials der allgemeinen Formel II in Gegenwart von sauren Verbindungen mit Wasser und/oder einem Alkanol umsetzt. Besonders vorteilhaft gestaltet sich das Verfahren, wenn man die 4-Monoacetale des (E)-2-Methyl-2-buten-1,4-dials der allgemeinen Formel I aus dem erhaltenen Reaktionsgemisch destillativ abtrennt, das hierbei zusätzlich anfallende Gemisch aus 2-Methyl-2-buten-1,4-dial und dessen Mono- und Bisacetalen in an sich bekannter Weise zu Bisacetalen der allgemeinen Formel II umsetzt und diese erneut der erfindungsgemäßen Umsetzung zuführt.

Dieser Befund ist überraschend, da nicht vorauszusehen war, daß bei der Umsetzung von 1-Monoacetalen des 2-Methyl-2-buten-1,4-dials mit Wasser und Alkoholen und von 2-Methyl-2-buten-1,4-dial mit Alkoholen ganz überwiegend 4-Monoacetale des 2-Methyl-2-buten-1,4-dials entstehen.

Es war auch aufgrund von EP-PS 9 752 nicht vorauszusehen, daß sich Bisacetale des 2-Methyl-2-buten-1,4-dials, in denen beide Acetalgruppen gleich sind, nahezu selektiv zu 4-Monoacetalen des 2-Methyl-2-buten-1,4-dials umsetzen lassen würden.

Weiterhin war überraschend, daß sich die isomeren 1-Acetale und 4-Acetale des 2-Methyl-2-buten-1,4-dials destillativ trennen lassen.

Als Ausgangsverbindungen der Formel II sind z.B. die 1-Mono- oder 1,4-Bisacetale des 2-Methylfumardialdehyds in Form der Dimethyl-, Diethyl-, Di-n-propyl-, Di-n-butyl, Diisobutyl-, Dicyclohexyl-, Dicyclopentyl- und Dibenzylacetale geeignet. Als entsprechende cyclische Acetale kommen z.B. solche ausgehend von Ethylenglykol, Propylenglykol oder Neopentylglykol als zweiwertiger Alkoholkomponente in Frage. Weiterhin ist 2-Methylfumardialdehyd selbst geeignet. Es ist auch möglich, Gemische der genannten Verbindungen zu verwenden, in denen auch die entsprechenden (Z)-Isomeren der Verbindungen II enthalten sein können.

Als 1-Mono- oder 1,4-Bisacylale des 2-Methylfumardialdehyds kommen z.B. Verbindungen in Frage, denen die Carbonsäuren Ameisensäure, Essigsäure, Propionsäure, Buttersäure, Cyclohexancarbonsäure, Phenylessigsäure zugrunde liegen.

Die Herstellung von (E)-2-Methyl-4,4-dimethoxy-2-butenal durch Umsetzung von 2-Methyl-1,1,4,4-tetramethoxy-2-buten mit Wasser in Gegenwart eines stark sauren Ionenaustauschers läßt sich z.B. durch die folgende Reaktionsgleichung beschreiben:

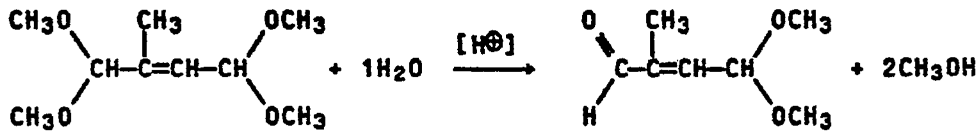

Als Nebenkomponenten treten hierbei unumgesetztes Ausgangsprodukt, 3-Methyl-4,4-dimethoxy-2-butenal und 2-Methyl-2-buten-1,4-dial auf. Ein besonderer Vorteil des Verfahrens besteht darin, daß man zunächst das gewünschte 2-Methyl-4,4-dimethoxy-2-butenal destillativ von den genannten Nebenkomponenten abtrennen, die anfallenden Gemische aus 2-Methyl-2-buten-1,4-dial und dessen Mono- und Bisacetalen mit Acetalisierungsmitteln, wie Alkoholen oder Orthoestern, in bekannter Weise wieder zu 2-Methyl-1,1,4,4-tetramethoxy-2-buten umsetzen und dieses erneut für die Herstellung von 2-Methyl-4,4-dimethoxy-2-butenal verwenden kann.

Die Ausgangsverbindungen der Formel II sind auf unterschiedlichen Wegen zugänglich. So kann man z.B. 2-Methyl-2-buten-1,4-dial durch Hydrolyse von 2-Methyl-4,4-diacetoxy-2-butenal herstellen (vgl. EP-PS 90 231). 3-Methyl-4,4-dimethoxy-2-butenal ist beispielsweise durch Oxidation von 3-Methyl-4,4-dimethoxy-2-butenol zugänglich. 2-Methyl-1,1,4,4-tetramethoxy-2-buten läßt sich durch Umsetzung von 2-Methyl-2-buten-1,4-dial oder 3-Methyl-4,4-dimethoxy-2-butenal mit Methanol oder o-Ameisensäuretrimethylester oder durch thermische Zersetzung des Bis-Dimethylacetals des 1,2-Diformylcyclopropans herstellen.

Als Verbindungen der Formel III können beispielsweise Wasser, Methanol, Ethanol, n- und i-Propanol, n-Butanol, 2-Ethyl-hexanol, n-Octanol, Cyclohexanol, Cyclopentanol, Benzylalkohol, Ethylenglykol, 1,2- und 1,3-Propylenglykol, 2-Methyl-1,3-propylenglykol, 2,2-Dimethyl-1,3-propylenglykol (Neopentylglykol) in Frage. Es ist auch möglich, Gemische aus Wasser und einem der angegebenen Alkohole zu verwenden.

Das Molverhältnis von Ausgangsverbindung II zur Verbindung III beträgt eins zu zwanzig, insbesondere eins zu fünf.

Als sauer wirkende Mittel verwendet man z.B. aliphatische Carbonsäuren, wie Ameisensäure, Essigsäure, Propionsäuren oder Valeriansäuren, Sulfonsäuren, wie p-Toluolsulfonsäure, Mineralsäuren wie Salzsäure, Schwefelsäure oder Bromwasserstoffsäure. Weiterhin lassen sich auch Kationenaustauscher in ihrer Säureform verwenden, z.B. Austauscher, die aus Styrol und Divinylbenzol aufgebaut sind und Sulfonsäuregruppen enthalten, oder anorganische Kationenaustauscher, wie Zeolithe, Phenol- oder Polystyrolsulfonsäureharze, Styrolphosphonsäureharze, Styrolphosphinsäureharze, oder entsprechende saure Harze enthaltende Austauscher, z.B. bifunktionelle Kondensationsharze.

Kationenaustauscher der genannten Art sind z.B. die im Handel unter den Bezeichnungen Lewatit® S 100, Amberlit® IR-200, Lewasorb®, Dowex® 50 WX 8, Amberlyst® 15 erhältlichen Produkte. Als sauer wirkende Verbindungen kann man auch Lewissäuren, wie Bortrifluorid, Zinkchlorid, Eisen(III)chlorid, Aluminiumchlorid oder Zinn(IV)chlorid verwenden.

Die Carbonsäuren der genannten Art werden z.B. in Mengen von 0,01 bis 1 Molen pro Mol der Ausgangsstoffe II angewendet. Bei Verwendung der Sulfonsäuren oder der starken Mineralsäuren und Lewissäuren genügen katalytische Megnen, wie 0,002 bis 0,25 Äquivalente der Säuren pro Mol der Verbindung II. Die Menge der Kationenaustauscher hängt von der Selektivität bzw. der Zahl der austauschaktiven Gruppen der verwendeten Austauscher bei der Reaktionstemperatur ab. Im allgemeinen verwendet man 1 bis 40 Gewichtsprozent, vorzugsweise 1 bis 25 Gewichtsprozent Austauscher, bezogen auf die Ausgangsstoffe II.

Die Umsetzung kann bei Temperaturen von 20 bis 200°C, insbesondere 40 bis 150°C, drucklos oder unter Druck, diskontinuierlich oder kontinuierlich durchgeführt werden.

Die Acetalisierung von Mono- oder Dialdehyden kann nach bekannten Verfahren, beispielsweise durch Umsetzung von Alkoholen oder Orthoestern durchgeführt werden (vgl. Houben-Weyl, Methoden der organischen Chemie, 4. Auflage, Band 6/3, Seiten 221-229).

Das erfindungsgemäße Verfahren kann beispielsweise so durchgeführt werden, daß man die Ausgangsverbindungen II, z.B. ein Bisacetal des 2-Methyl-2-buten-1,4-dials, mit der jeweiligen Menge Wasser und z.B. einem starksauren Ionenaustauscher als saurer Verbindung auf die gewünschte Temperatur erhitzt. Es ist auch möglich, den gebildeten Alkohol während der Umsetzung abzudestillieren. Nach der Umsetzung wird das Reaktionsgemisch abgekühlt, der Ionenaustauscher abfiltriert und das gewünschte 4-Monoacetal des 2-Methyl-2-buten-1,4-dials durch fraktionierende Destillation abgetrennt. Das dabei außerdem erhaltene Gemisch aus 2-Methyl-2-buten-1,4-dial, Monoaldehyd und Bisacetal wird durch Umsetzung mit einem Acetalisierungsmittel in das jeweilige Bisacetal zurückverwandelt, das erneut für die erfindungsgemäße Umsetzung einsetzbar ist.

Es ist auch möglich, in Gegenwart von unter den Reaktionsbedingungen inerten Lösungsmitteln wie Kohlenwasserstoffen, chlorierten Kohlenwasserstoffen oder aromatischen Kohlenwasserstoffen zu arbeiten.

Die 4-Acetale des 2-Methyl-2-buten-1,4-dials stellen wertvolle Zwischenprodukte dar, die z.B. für die Synthese von Terpenen Verwendung finden (vgl. DE-OS 23 57 810). Mit Hilfe des erfindungsgemäßen Verfahren können sie auf recht einfache Weise und ohne Verwendung von teuren und/oder toxischen Oxidationsmitteln hergestellt werden.

Beispiel 1

Eine Suspension von 1 g Amberlite IR 120 (H-Form) in 47,6 g (E)-2-Methyl-1,1,4,4-tetramethoxy-2-buten(1) und 5 g Wasser wurde auf 60°C erwärmt und 0,5 Stunden (h) bei dieser Temperatur gerührt. Nach dem Abkühlen auf Raumtemperatur (RT) und Abfiltrieren des Ionenaustauschers enthielt der Reaktionsaustrag laut quantitativer gaschromatographischer Analyse 7,6 g Ausgangsprodukt, 22,8 g 2-Methyl-4,4-dimethoxy-2-butenal(2), 5,5 g 3-Methyl-4,4-dimethoxy-2-butenal(3), 1,5 g 2-Methyl-2-buten-1,4-dial(4) und 0,5 g 3-Methyl-2,5-dimethoxy-2,5-dihydrofuran(5). Das Verhältnis von 2-Methyl- zu 3-Methyl-4,4-dimethoxy-2-butenal betrug also 81 zu 19.

Zur Abtrennung von 2-Methyl-4,4-dimethoxy-2-butenal wurde der Reaktionsaustrag an einer 1 m-Drehbandkolonne destilliert. Die Zusammensetzung der dabei erhaltenen 7 Fraktionen ist in der folgenden Tabelle 1 angegeben.

Tabelle 1

| Fraktion | Menge [g] | Siedepunkt [°C/18 mbar] | GC-Analyse (F1.-%) (1) | (2) | (3) | (4) | (5) |
|---|---|---|---|---|---|---|---|
| 1 | 1,5 | 47–75 | – | 6,7 | – | 47,6 | 45,0 |
| 2 | 1,4 | 75–77 | – | 55,7 | – | 28,1 | 15,8 |
| 3 | 8,9 | 77–80 | – | 95,6 | – | 2,7 | 1,7 |
| 4 | 11,4 | 80–81 | – | 99,0 | – | 0,1 | 0,2 |
| 5 | 0,8 | 81–86 | 1,0 | 88,3 | 6,1 | – | – |
| 6 | 5,8 | 86–92 | 6,3 | 9,3 | 80,7 | – | – |
| 7 | 6,0 | 92–95 | 54,4 | 0,3 | 39,2 | – | – |

Der Destillationsrückstand betrug 0,5 g, in der Kühlfalle waren 16,3 g Methanol enthalten. Die Ausbeute an (E)-2-Methyl-4,4-dimethoxy-2-butenal betrug 20,5 g (57 %, bezogen auf eingesetztes (1) unter der Annahme, daß Fl.-% = Gew.% sind).

Die Fraktionen 1, 2, 6 und 7 wurden vereinigt (14,7 g), mit 21,2 g o-Ameisensäuretrimethylester und 0,5 g Amberlite IR-120 versetzt und 20 h bei RT gerührt. Nach dem Abziehen von Leichtsiedern am Rotationsverdampfer wurden durch Destillation 14,4 g (E)-2-Methyl-1,1,4,4-tetramethoxy-2-buten zurückgewonnen.

Die Selektivität bei der Herstellung von (E)-2-Methyl-4,4-dimethoxy-2-butenal betrug demnach 82 %.

Beispiel 2

Eine Lösung von 0,9 g Wasser in 36 g E-3-Methyl-4,4-dimethoxy-2-butenal und 16 g Methanol, in der 1 g Amberlite IR-120 suspendiert war, wurde auf 60°C erwärmt und 0,5 h bei dieser Temperatur gerührt. Nach dem Abkühlen auf RT und Abfiltrieren des Ionenaustauschers enthielt der Reaktionsaustrag laut quantitativer gaschromatographischer Analyse 1,9 g 2-Methyl-1,1,4,4-tetramethoxy-2-buten (1), 21,8 g 2-Methyl-4,4-dimethoxy-2-butenal (2), 5,3 g Ausgangsprodukt (3), 7,0 g 2-Methyl-2-buten-1,4-dial (4) und 0,4 g 3-Methyl-2,5-dimethoxy-2,5-dihydrofuran (5). Das Verhältnis von 2-Methyl zu 3-Methyl-4,4-dimethoxy-2-butenal betrug demnach 80 zu 20.

Wie in Beispiel 1 beschrieben, wurde der Reaktionsaustrag an einer Drehbandkolonne destilliert. Die Zusammensetzung der dabei erhaltenen Fraktionen ist in der folgenden Tabelle 2 angegeben.

Tabelle 2

| Fraktion | Menge [g] | Siedepunkt [°C/18 mbar] | GC-Analyse (F1.-%) (1) | (2) | (3) | (4) | (5) |
|---|---|---|---|---|---|---|---|
| 1 | 2,5 | 35–76 | – | 22,9 | – | 48,3 | 28,2 |
| 2 | 21,3 | 76–80 | – | 91,0 | 1,7 | 3,1 | 1,5 |
| 3 | 11,0 | 80–90 | 18,9 | 10,4 | 68,3 | – | – |

Die Ausbeute am (E)-2-Methyl-4,4-dimethoxy-2-butenal betrug 20,5 g (57 %, bezogen auf eingesetztes (3)). Der Destillationsrückstand wog 1,5 g in der Kühlfalle waren 19 g Methanol enthalten.

Beispiel 3

Eine Lösung von 1 g 2-Methyl-2-buten-1,4-dial in 6 g Methanol, in der 0,2 g des Ionenaustauschers Amberlite IR-120 suspendiert waren, wurde 5 Minuten lang auf 55°C erhitzt. Nach dem Abkühlen auf RT und Abfiltrieren des Ionenaustauschers wurde das Reaktionsgemisch gaschromatographisch analysiert. Es bestand zu 53 % aus E-2-Methyl-4,4-dimethoxy-2-butenal, zu 8 % aus (E)-3-Methyl-4,4-dimethoxy-2-butenal, zu 10 % aus 2-Methyl-2-buten-1,4-dial und zu 20 % aus (E)-2-Methyl-1,1,4,4-tetramethoxy-2-buten.

Beispiel 4

Eine Lösung von 0,2 g konzentrierter Schwefelsäure und 1 g Wasser in 9,5 g 2-Methyl-1,1,4,4-tetramethoxy-2-buten wurde 0,5 h bei RT gerührt. Nach dem Neutralisieren der Schwefelsäure mit festem $NaHCO_3$ wurde filtriert. Das Filtrat wurde durch Kugelrohrdestillation aufgearbeitet (150°C/20 mbar). Hierbei wurden 0,8 g Rückstand und 4,8 g Destillat gewonnen. Die gaschromatographische Analyse ergab 55,6 % (E)-2-Methyl-4,4-dimethoxy-2-butenal, 2,9 % 2-Methyl-2-buten-1,4-dial, 18,8 % 3-Methyl-2,5-dimethoxy-2,5-dihydrofuran und 8,2 % unumgesetztes Ausgangsprodukt.

Beispiel 5

Eine Lösung von 24,5 g 2-Methyl-2-buten-1,4-dial in 24 g Methanol, in der 1 g Amberlite IR-120 suspendiert war, wurde auf 60°C erwärmt und 0,5 h bei dieser Temperatur gerührt. Nach dem Abkühlen auf RT und Abfiltrieren des Ionenaustauschers wurde das Reaktionsgemisch gaschromatographisch untersucht. Die quantitative Analyse zeigte, daß 41,4 % (E)-2-Methyl-4,4-dimethoxy-2-butenal (2), 11,3 % (E)-3-Methyl-4,4-dimethoxy-2-butenal (3), 16,8 % Ausgangsprodukt (4), 1,4 % 2-Methyl-1,1,4,4-tetramethoxy-2-buten (1) und 1,6 % 3-Methyl-2,5-dimethoxy-2,5-dihydrofuran (5) enthalten waren.

Wie in Beispiel 1 beschrieben, wurde der Reaktionsaustrag an einer Drehbandkolonne destilliert. Die Zusammensetzung der dabei erhaltenen Fraktionen ist in der folgenden Tabelle 3 angegeben.

Tabelle 3

| Fraktion | Menge [g] | Siedepunkt [°C/18 mbar] | GC-Analyse (F1.-%) (1) | (2) | (3) | (4) | (5) |
|---|---|---|---|---|---|---|---|
| 1 | 7,1 | 67–68 | – | 1,3 | – | 75,7 | 22,3 |
| 2 | 2,2 | 68–75 | – | 17,7 | 0,8 | 73,1 | 5,8 |
| 3 | 17,8 | 75–86 | – | 94,6 | 2,2 | 2,1 | – |
| 4 | 5,2 | 86–90 | 4,3 | 11,4 | 81,1 | – | – |

Die Ausbeute an (E)-2-Methyl-4,4-dimethoxy-2-butenal betrug 16,8 g (47 %, bezogen auf eingesetztes (4)). Der Destillationsrückstand wog 1,7 g; in der Kühlfalle waren 16,4 g Methanol enthalten.

Beispiel 6

Eine Suspension von 0,1 g Amberlite IR 120 in 0,6 g Wasser und 2,1 g des Bisacetals aus 2-Methyl-2-buten-1,4-dial und Neopentylglykol wurde 0,5 h lang bei 100 ± 2°C gerührt. Die gaschromatographische Analyse des abgekühlten und filtrierten Reaktionsgemisches zeigte, daß es zu 20,8 % aus unumgesetztem Bisacetal, zu 21,4 % aus dem 4-Monoacetal, zu 12,3 % aus dem 1-Monoacetal und zu 31,4 % aus Neopentylglykol bestand. Das Verhältnis von 4-Monoacetal zu 1-Monoacetal betrug demnach 64:36.

## Patentansprüche

1. Verfahren zur Herstellung von 4-Monoacetalen des (E)-2-Methyl-2-buten-1,4-dials der allgemeinen Formel I

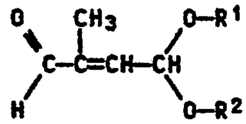

(I),

in der $R^1$ und $R^2$ einen aliphatischen Kohlenwasserstoffrest mit 1 bis 12 C-Atomen, einen Cycloalkylrest mit 5 bis 7 C-Atomen oder eine Benzylgruppe bedeuten, oder aber $R^1$ und $R^2$ zusammen für Ethylen- oder Propylenreste stehen, die noch durch Alkylgruppen mit 1 bis 4 C-Atomen, substituiert sein können, dadurch gekennzeichnet ist, daß man Verbindungen der allgemeinen Formel II

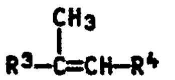

(II),

in der $R^3$ und $R^4$ entweder beide für eine der Gruppen

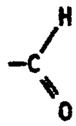

,

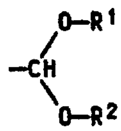

oder

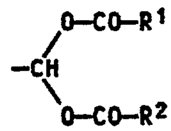

stehen, in der $R^1$ und $R^2$ die oben angegebene Bedeutung haben, oder aber $R^3$ eine der Gruppen

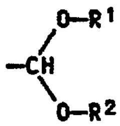

oder

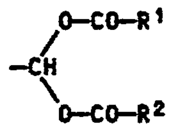

und $R^4$ die Formylgruppe

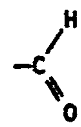

bedeuten, bei Temperaturen von 20 bis 200°C, vorzugsweise 40 bis 150°C in Gegenwart von sauren Verbindungen mit Verbindungen der Formel III

$R^5$-OH(III),

in der $R^5$ für Wasserstoff oder einen Alkylrest mit 1 bis 12 C-Atomen oder einen Hydroxyalkylrest mit 2 bis 5 C-Atomen bedeutet, umsetzt, wobei im Fall, daß $R^3$ und $R^4$ für

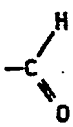

stehen, $R^5$ einen Alkylrest bedeutet und für den Fall, daß $R^3$ und $R^4$ für

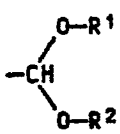

oder

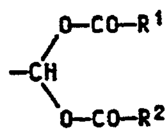

stehen, $R^5$ Wasserstoff bedeutet und für den Fall, daß $R^3$ für

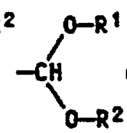

oder

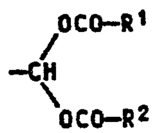

und $R^4$ für

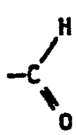

stehen, $R^5$ Wasserstoff und/oder einen der Alkylreste bedeutet.

2. Verfahren zur Herstellung von 4-Monoacetalen des (E)-2-Methyl-2-buten-1,4-dials der allgemeinen Formel I gemäß Anspruch 1, dadurch gekennzeichnet, daß man ein 1,4-Bisacetal der Formel II in Gegenwart von sauren Verbindungen mit Wasser umsetzt.

3. Verfahren zur Herstellung von 4-Monoacetalen des (E)-2-Methyl-2-buten-1,4-dials der allgemeinen Formel I gemäß Anspruch 1, dadurch gekennzeichnet, daß man 2-Methyl-2-buten-1,4-dial in Gegenwart von sauren Verbindungen mit Alkanolen umsetzt.

4. Verfahren zur Herstellung von 4-Monoacetalen des (E)-2-Methyl-2-buten-1,4-dials der allgemeinen Formel I gemäß Anspruch 1, dadurch gekennzeichnet, daß man 1-Monoacetale des (E)-2-Methyl-2-buten-1,4-dials der allgemeinen Formel I in Gegenwart von sauren Verbindungen mit Wasser und/oder einem Alkanol umsetzt.

5. Verfahren gemäß Anspruch 1, dadurch gekennzeichnet, daß man die 4-Monoacetale des (E)-2-Methyl-2-buten-1,4-dials der allgemeinen Formel I aus dem erhaltenen Reaktionsgemisch destillativ abtrennt, das hierbei zusätzlich anfallende Gemisch aus 2-Methyl-2-buten-1,4-dial und dessen Mono- und Bisacetalen in an sich bekannter Weise zu Bisacetalen der allgemeinen Formel II umsetzt und diese erneut der erfindungsgemäßen Umsetzung zuführt.

**Claims**

1. A process for the preparation of a 4-monoacetal of (E)-2-methylbut-2-ene-1,4-dial of the formula I

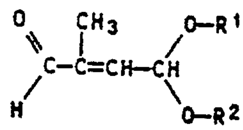

(I)

where $R^1$ and $R^2$ are each aliphatic hydrocarbyl of 1 to 12 carbon atoms, cycloalkyl of 5 to 7 carbon atoms or benzyl, or $R^1$ and $R^2$ together form an ethylene or propylene radical which may be further substituted by alkyl of 1 to 4 carbon atoms, wherein a compound of the formula II

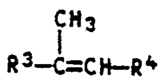

(II)

where either $R^3$ and $R^4$ are both one of the groups

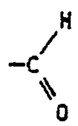 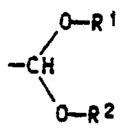 or 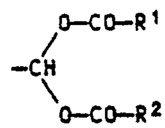

where $R^1$ and $R^2$ have the above meanings, or $R^3$ is one of the groups

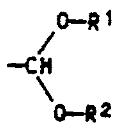 or 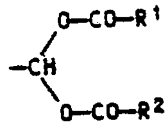

and $R^4$ is formyl

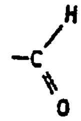

is reacted, at from 20 to 200°C, preferably 40 to 150°C, in the presence of an acidic compound, with a compound of the formula III

$R^5$–OH (III)

where $R^5$ is hydrogen, alkyl of 1 to 12 carbon atoms or hydroxyalkyl of 2 to 5 carbon atoms, and, where $R^3$ and $R^4$ are each

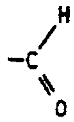 $R^5$ is alkyl, and where $R^3$ and $R^4$ are each

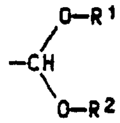 or 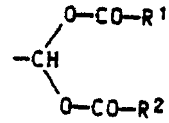, $R^5$ is hydrogen, and where

$R^3$ - is 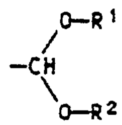 or 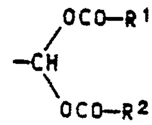 and $R^4$ is 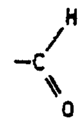

$R^5$ is hydrogen and/or alkyl.

2. A process for the preparation of a 4-monoacetal of (E)-2-methylbut-2-ene-1,4-dial of the formula I as claimed in claim 1, wherein a 1,4-bisacetal of the formula II is reacted with water in the presence of an acidic compound.

3. A process for the preparation of a 4-monoacetal of (E)-2-methylbut-2-ene-1,4-dial of the formula I as claimed in claim 1, wherein 2-methylbut-2-ene-1,4-dial is reacted with an alkanol in the presence of an acidic compound.

4. A process for the preparation of a 4-monoacetal of (E)-2-methylbut-2-ene-1,4-dial of the formula I as claimed in claim 1, wherein a 1-monoacetal of (E)-2-methylbut-2-ene-1,4-dial of the formula I is reacted with water and/or an alkanol in the presence of an acidic compound.

5. A process as claimed in claim 1, wherein the 4-monoacetal of (E)-2-methylbut-2-ene-1,4-dial of the formula I is isolated from the resulting reaction mixture by distillation, the resulting additional mixture of 2-methylbut-2-ene-1,4-dial and its mono- and bisacetals is converted in a conventional manner to a bisacetal of the formula II, and the latter is recycled to the novel reaction.

**Revendications**

1. Procédé de préparation de 4-monoacétals du (E)-2-méthyl-2-buten-1,4-dial de la formule générale I.

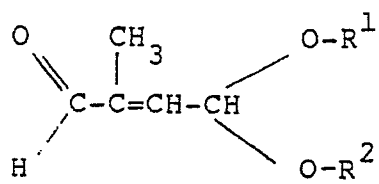 (I)

dans laquelle $R^1$ et $R^2$ représentent un reste d'hydrocarbure aliphatique de 1 à 12 atomes C, un reste cycloalkyle de 5 à 7 atomes C ou un groupe benzyle, ou bien $R^1$ et $R^2$ ensemble sont mis pour de restes éthylène ou propylène qui peuvent encore être substitués par des groupes alkyle de 1 à 4 atomes C, caractérisé par le fait que, à des températures de 20 à 200°C de préférence 40 à 150°C, en présence de composés acides, l'on fait réagir des composés de formule générale II

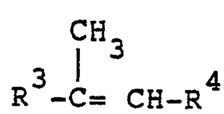 (II)

dans laquelle $R^3$ et $R^4$ ou bien représentent chacun un des groupes

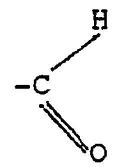 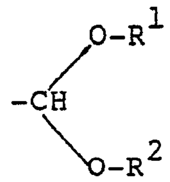 ou 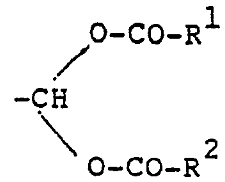

dans lesquels $R^1$ et $R^2$ ont les significations sus-indiquées, ou bien $R^3$ représente un des groupes

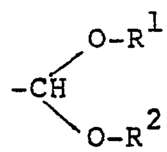 ou 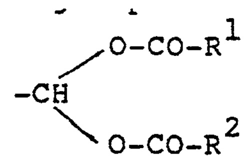

et $R^4$ le groupe formyle

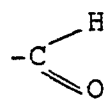

avec des composés de formule

$R^5$–OH (III)

dans laquelle $R^5$ représente hydrogène ou un reste alkyle de 1 à 12 atomes C ou un reste hydroxyalkyle de 2 à 5 atomes C.

$R^5$ représentant un reste alkyle, dans le cas où $R^3$ et $R^4$ représentent

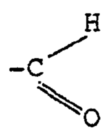 , $R^5$ représentant hydrogène, dans le cas où $R^3$ et $R^4$ représentent

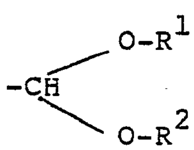 ou 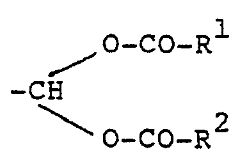 et $R^5$ représentant hydrogène

et/ou un des restes alkyle, dans le cas où $R^3$ représente

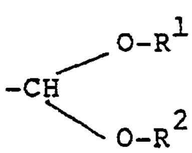 ou 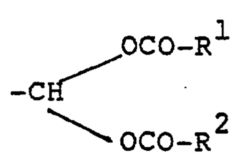 et $R^4$ représente 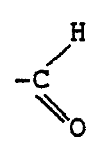

2. Procédé de préparation de 4-monoacétals du (E)-2-méthyl-2-buten-1,4-dial de formule générale I selon la revendication 1, caractérisé par le fait que l'on fait réagir avec de l'eau un 1,4-bisacetal de formule II en présence de composés acides.

3. Procédé de préparation de 4-monoacétals du (E)-2-méthyl-2-buten-1,4-dial de formule générale I selon la revendication 1, caractérisé par le fait que l'on fait réagir des alcanols du 2-méthyl-2-buten-1,4-dial en présence de composés acides.

4. Procédé de préparation de 4-monoacétals du (E)-2-méthyl-2-buten-1,4-dial de formule général I selon la revendication 1, caractérisé par le fait que l'on fait réagir avec de l'eau et/ou un alcanol du 1-monoacétal du (E)-2-méthyl-2-buten-1,4-dial de formule général I présence de composés acides.

5. Procédé selon la revendication 1, caractérisé par le fait que l'on sépare, par distillation, du mélange de réaction obtenu les 4-monoacétals du (E)-2-méthyl-2-buten-1,4-dial de formule générale I, que l'on transforme par réaction, de manière connue en soi, le mélange, obtenu ici additionnellement, de 2-méthyl-2-buten-1,4-dial et ses mono- et bisacetals en biosacetals de formule générale II, et on soumet à nouveau ceux-ci à la réaction selon l'invention.